(19) **Europäisches Patentamt / European Patent Office / Office européen des brevets**

(11) **EP 1 787 678 A2**

(12) ## EUROPÄISCHE PATENTANMELDUNG

(43) Veröffentlichungstag:
23.05.2007 Patentblatt 2007/21

(51) Int Cl.:
*A61N 2/02* (2006.01)

(21) Anmeldenummer: 06025428.1

(22) Anmeldetag: 31.05.2002

(84) Benannte Vertragsstaaten:
**AT BE CH CY DE DK ES FI FR GB GR IE IT LI LU MC NL PT SE TR**
Benannte Erstreckungsstaaten:
**AL LT LV MK RO SI**

(30) Priorität: **31.05.2001 DE 20109058 U**

(62) Dokumentnummer(n) der früheren Anmeldung(en) nach Art. 76 EPÜ:
**02735397.8 / 1 390 102**

(71) Anmelder: **Muntermann, Axel**
**35580 Wetzlar-Nauborn (DE)**

(72) Erfinder: **Muntermann, Axel**
**35580 Wetzlar-Nauborn (DE)**

(74) Vertreter: **Herden, Andreas F.**
**Blumbach - Zinngrebe**
**Patentanwälte**
**Alexandrastrasse 5**
**65187 Wiesbaden (DE)**

Bemerkungen:
•Diese Anmeldung ist am 08 - 12 - 2006 als Teilanmeldung zu der unter INID-Kode 62 erwähnten Anmeldung eingereicht worden.
•A request for correction (numierung Ansprüche) has been filed pursuant to Rule 88 EPC. A decision on the request will be taken during the proceedings before the Examining Division (Guidelines for Examination in the EPO, A-V, 3.).

(54) **Vorrichtung und Verfahren zur Behandlung mit magnetischen Feldern**

(57) Um eine gut transportierbare und lagerbare sowie eine für den Patienten bequeme Vorrichtung zur Behandlung mit magnetischen Feldern zur Verfügung zu stellen, welche insbesondere auch preiswert herstellbar ist, sieht die Erfindung eine Vorrichtung zur Behandlung mit magnetischen Feldern vor, welche:

eine erste Einrichtung zur Erzeugung eines ersten Magnetfeldes,

eine zweite Einrichtung zur Erzeugung eines zweiten Magnetfeldes, und

einen Träger, insbesondere eine Matte mit einer Oberseite und einer Unterseite umfaßt, wobei die Matte zum Auflegen von zu behandelnden Körperbereichen eines Patienten ausgebildet ist.

Fig. 1a

Fig. 1b

**Beschreibung**

Gebiet der Erfindung

Die Erfindung betrifft eine Vorrichtung und ein Verfahren zur Behandlung mit magnetischen Feldern im allgemeinen und zur Beeinflussung von Spins und/oder magnetischen Momenten in zu behandelndem Gewebe im speziellen.

Hintergrund der Erfindung

Nicht-invasive Behandlungsverfahren erobern immer neue Anwendungsgebiete in der Medizin. In Bezug auf die hier angemeldete Erfindung sind Vorrichtungen und Verfahren zur therapeutischen Behandlung mittels externer magnetischer Felder besonders hervorzuheben. Obwohl bisher die genaue Wirkungsweise solcher Therapien nicht bis ins Detail verstanden wurde, sind ihre therapeutischen Erfolge wissenschaftlich bewiesen und allgemein anerkannt. Untersuchungen zu Ergebnissen bekannter Magnetfeldtherapien finden sich z.B. in "Orthopädische Praxis" 8/2000, 36. Jahrgang, Seiten 510 bis 515 und in Fritz Lechner, "Elektrostimulation und Magnetfeldtherapie. Anwendung, Ergebnisse und Qualitätssicherung" 1989.

Insbesondere wurde in solchen Untersuchungen festgestellt, dass Magnetfeldtherapien bei den Patienten zum Teil deutliche Verbesserungen im Beschwerdebild bewirken ohne im wesentlichen nachweisbare negative Nebenwirkungen zu erzeugen. Ein weiterer großer Vorteil von Magnetfeldtherapien ist es, daß eine für den Patienten mit erheblichen Schmerzen, Risiken und Kosten verbundene Operation möglicherweise vollständig vermieden werden kann.

So ist z.B. aus der DE 40 26 173 eine Vorrichtung bekannt, welche gepulste und modulierte Magnetfelder erzeugt, um Patienten zu behandeln. Dabei wird Körpergewebe einem Magnetfeld ausgesetzt, welches sich als Überlagerung eines konstanten Magnetfeldes und eines magnetischen Wechselfeldes ergibt.

Gepulste Magnetfelder werden typischerweise mittels eines gepulsten Stromes, welcher durch eine Spule fließt erzeugt. Solche pulsierenden Felder in Spulen benötigen jedoch viel Energie und sind träge, da die Spuleninduktivität die Feldänderung verlangsamt.

Die Heilwirkung dieser Magnetfeldtherapie besteht unter anderem in der Linderung von Osteoporose oder den Folgen eines Schlaganfalls. Dabei erschien es wahrscheinlich, daß durch die angewandten Magnetfelder, Transport- und/oder Stoffwechselprozesse gefördert werden, die zu einer positiven therapeutischen Wirkung führen. Bislang ging man davon aus, daß die positive therapeutische Wirkung durch einen Energieaustausch zwischen Feldern und Bestandteilen von Zellen (Protonen, Ionen etc.) verursacht wird. Hierbei wurde die Energieübertragung durch die Anregung bzw. die Absorption von Ionen-Zyklotron-Resonanzen (ICR) in einem biologischen Körper erklärt und daher nach entsprechenden ICR-Bedingungen gesucht. Konsequenter Weise sind die bekannten Vorrichtungen auf die Erzeugung von ICR-Bedingungen ausgerichtet.

Allerdings erscheint diese Ursachenerklärung unter Umständen fraglich, da Zyklotronresonanzen im allgemeinen nur an freien Teilchen auftreten, wie beispielsweise im Vakuum oder bei Elektronen im Leitungsband eines Halbleiters. Ferner kann auch durch einfache Rechnung gezeigt werden, daß sich eine Zyklotron-Bewegung auf einer Kreisbahn vollziehen würde, deren Radius bereits den durchschnittlichen Durchmesser eines Querschnitts eines menschlichen Körpers übersteigt. Dies bedeutet, daß für den Energieübertrag eine Erklärung hinsichtlich einer Zyklotronresonanz insbesondere bei festem Gewebe fraglich sein kann.

Es ist auch denkbar, dass die Wirkung auf piezoelektrischen Vorgängen im Körper beruht. Dieser Erklärungsansatz geht davon aus, dass um jedes Körpergelenk herum ein elektrisches Feld besteht und im gesunden Zustand jede Bewegung eine Piezospannung verursacht, da der Knorpel piezoelektrische Eigenschaften besitzt. Im kranken Zustand könnten durch induzierte Spannungen diese Piezospannungen simuliert werden. Siehe hierzu auch Christian Thuile, "Das große Buch der Magnetfeldtherapie", Linz 1997.

Eine weitere Vorrichtung zur Behandlung eines biologischen Körpers mit magnetischen Feldern, welche Spinresonanzen innerhalb des zu behandelnden Körpers erzeugt, ist aus der Offenlegungsschrift WO 99/66986 desselben Anmelders bekannt. Diese in der Offenlegungsschrift WO 99/66986 beschriebene Vorrichtung ist allerdings im wesentlichen darauf ausgerichtet eine gezielte reproduzierbare Behandlung mit Magnetfeldern in allen biologischen Materialien vorzunehmen, unabhängig davon, ob ionische Teile vorhanden sind. Mit der genannten Vorrichtung werden die positiven therapeutischen Wirkungen durch die Erzeugung von Spinresonanzen und Spinresonanzsequenzen erzielt. Hierbei wird die Kernspinresonanz aber insbesondere auch zur Energieübertragung eingesetzt.

Auf anderen Gebieten der Technik sind Kernspinresonanzverfahren (sogenannte NMR-Verfahren) bereits seit langem bekannt. Sie werden insbesondere in der medizinischen Diagnostik und allgemein für die hochpräzise Magnetfeldmessung verwendet. Zu letzterer Anwendung sei beispielhaft das "Virginia Scientific FW101 Flowing Water NMR Teslameter" genannt. Eine Beschreibung dieses Gerätes findet sich unter www.gmw.com/magnetic_measurements/VSI/FW101.html

Es bleibt festzuhalten, dass die bekannten Vorrichtungen der therapeutischen Medizin zumeist große Spulensysteme umfassen mit denen die Magnetfelder generiert und geändert werden. Diese Spulensysteme weisen aber eine hohe Induktivität auf, was zu großen Schaltzeitkonstanten und hohem Energieverbrauch führt. Große Schaltzeiten führen aber nachteiligerweise zu einer geringen Effizienz bezüglich dynamischer Vorgänge im Körper.

Ferner sind die Spulensysteme typischerweise derart ausgebildet, dass sie Öffnungen aufweisen, in welche Körperteile, z.B. Arme oder Beine eingeführt werden können. Hierdurch sind die bekannten Vorrichtungen insgesamt relativ unförmig und weisen Nachteile bezüglich deren Lagerungs- und Transportmöglichkeiten auf. Im übrigen sind sie teilweise unbequem für den Patienten. Weiter ist der Energiebedarf der meisten bekannten Vorrichtungen sehr hoch, da mittels der Spulensysteme starke Magnetfelder erzeugt werden.

Darüber hinaus verbleibt noch immer eine Reihe offener Fragen hinsichtlich der physikalisch-physiologischen Wirkungsweise der Vorrichtungen und der von ihnen im Körper ausgelösten Prozesse. Ohne die genaue Kenntnis der Wirkungsweise war es allerdings in der Vergangenheit nur schwer möglich einen optimierten Aufbau und die optimalen Parameter für dessen Betrieb zu bestimmen.

Daher ist es eine Aufgabe der vorliegenden Erfindung eine verbesserte Vorrichtung und ein verbessertes Verfahren zur Behandlung mit magnetischen Feldern zur Verfügung zu stellen.

Eine weitere Aufgabe der Erfindung ist es, eine Vorrichtung und ein Verfahren verfügbar zu machen, mit welchen im Körper durch Bewegung erzeugte elektromagnetische Reize, insbesondere das natürliche Verhalten von magnetischen Momenten im Körper bei Bewegung im Erdmagnetfeld künstlich nachbildbar bzw. simulierbar sind.

Noch eine Aufgabe der Erfindung ist es, eine Vorrichtung und ein Verfahren verfügbar zu machen, welche schnelle Schaltzeitkonstanten ermöglichen und einen geringen Energieverbrauch aufweisen.

Eine weitere Aufgabe der Erfindung ist es, eine gut transportierbare und lagerbare sowie eine für den Patienten bequeme Vorrichtung zur Behandlung mit magnetischen Feldern zur Verfügung zu stellen, welche insbesondere auch preiswert herstellbar ist.

Die Aufgabe der Erfindung wird auf überraschend einfache Weise bereits durch den Gegenstand der Ansprüche 1, 16 und 37 gelöst. Vorteilhafte und bevorzugte Weiterbildungen sind Gegenstand der abhängigen Ansprüche.

Der Erfindung liegt die höchst überraschende Erkenntnis zugrunde, dass positive therapeutische Wirkungen bei der Behandlung mit magnetischen Feldern auf eine Bewegungssimulation über Spinresonanzsignale zurückgeführt werden können.

In einem menschlichen, tierischen oder sonstigen biologischen Körper können sich magnetische Momente, z.B. Elektronen- oder Kernspinmomente bereits im Erdmagnetfeld ausrichten und erzeugen damit eine makroskopische Magnetisierung. Jede Bewegung eines Körperteils führt zu einer kleinen Änderung der Richtung dieser Magnetisierung. Solange die Magnetisierungsrichtung nicht parallel zur Erdmagnetfeldrichtung ausgerichtet ist, präzediert die Magnetisierung mit einer Frequenz von ca. 2000 Hz im Erdmagnetfeld und induziert in der Umgebung eine Wechselspannung mit der gleichen Frequenz. Diese induzierte Spannung kann man mit einer äußeren Spule messen, sie liegt im mV-Bereich. Im Körper aber ist die induzierte Spannung aufgrund der kleineren Abstände deutlich größer. Das menschliche Nervensystem registriert diese Spannung und erkennt so die Bewegung. Als Folge wird der Stoffwechsel aktiviert, da für die Muskelarbeit Energie benötigt wird.

Aufgrund verschiedener Krankheiten sind die Bewegung eines Patienten und dessen Stoffwechsel eingeschränkt. Mit der erfindungsgemäßen Vorrichtung und dem Verfahren wird eine vorbestimmte und gezielte Drehung der Spins bzw. der durch die Spins erzeugten makroskopischen Magnetisierung im Gewebe herbeigeführt. Es wird also hinsichtlich der natürlicherweise durch das Erdmagnetfeld im Körper erzeugten Spinresonanzen der Organismus bezüglich einer in Realität nicht stattfindenden Bewegung getäuscht. Hierzu erzeugt die erfindungsgemäße Vorrichtung geeignete magnetische Felder, welche die Ausrichtung der Spins und/oder der Magnetisierung in einer Weise verändern, dass eine Bewegung des im Behandlungsbereich angeordneten Körperbereichs simuliert wird. In diesem Zusammenhang konnte unter anderem durch die Anwendung der vorliegenden Erfindung ein sehr guter Behandlungserfolg bei der Therapie von Osteoporose festgestellt werden.

Eine erste Ausführungsform der Erfindung zeichnet sich dadurch aus, daß die erfindungsgemäße Vorrichtung zur Behandlung mit magnetischen Feldern eine erste und zweite Einrichtung zur Erzeugung eines ersten bzw. zweiten Magnetfeldes und einen Träger, insbesondere eine Matte zum Auflegen und/oder Anlegen von zu behandelnden Körperbereichen eines Patienten oder des ganzen Patienten umfaßt. Hierbei definiert der Träger, wie etwa die Matte eine Oberseite und eine Unterseite, zwischen welchen vorzugsweise die erste und zweite Einrichtung zur Erzeugung des ersten bzw. zweiten Magnetfeldes angeordnet sind. Diese Anordnung ermöglicht eine sehr kompakte, insbesondere sehr flache Bauweise.

Als Träger kommen neben einer Matte, in welcher die Einrichtungen zur Erzeugung des ersten bzw. zweiten Magnetfeldes angeordnet sind, auch beispielsweise eine Behandlungsliege oder ein Behandlungsstuhl in Frage. Auch Systeme, die an den Patienten, beziehungsweise an das zu behandelnde Gewebe angelegt werden, sind möglich. Beispielsweise kann der Träger eine mehrflügelige Anordnung umfassen, welche sich um einen Körperteil, insbesondere den Kopf eines Patienten legen läßt, welche an den Kopf eines Patienten angelegt wird. Diese Vorrichtung kann zum Beispiel mehrere Flügel umfassen, die derart abgemessen sind, daß sie sich um beide Ohren oder um den Kiefer eines Patienten legen lassen. Insbesondere können auch bei dieser Form des Trägers erste und zweite Einrichtungen zur Erzeugung des ersten bzw. zweiten Magnetfeldes in mehreren oder allen Flügeln integriert sein.

Weiterhin kann der Träger auch als Gamasche ausgebildet sein, welche sich beispielsweise um Beine oder Arme legen läßt.

Auch ein Träger, welcher eine Decke umfaßt, kann für bestimmte Anwendungen vorteilhaft sein. Für die Behandlung von Tieren etwa, wie unter anderem von Pferden kann die Decke zur Behandlung über das Tier gelegt werden.

Vorrichtung nach einem der Ansprüche 1 bis 14, dadurch gekennzeichnet, daß der Träger eine Behandlungsliege und/oder einen Behandlungsstuhl und/oder eine mehrflügelige Anordnung, welche sich um einen Körperteil, insbesondere den Kopf eines Patienten legen läßt, und/oder eine Gamasche und/oder eine Decke umfaßt.

Der Form und Beschaffenheit des Trägers sind, wie anhand der obige Beispiele deutlich wird, demnach kaum Grenzen gesetzt und kann dem Anwendungszweck entsprechend angepaßt werden.

Die Atomkerne im Patientengewebe definieren in den Magnetfeldern eine Spinresonanzfrequenz oder weisen eine solche auf. Dabei ist die Resonanzfrequenz mit der Feldstärke des Magnetfelds korreliert. Für Wasserstoffatome gilt beispielsweise die Gleichung

$$F[kHz] = 4,225 \times B \ [Gauss],$$

wobei F die Kernspinresonanzfrequenz in Kilohertz und B die Magnetfeldstärke in Gauss bezeichnen. Beispielsweise beträgt die Kernspinresonanzfrequenz 16,9 kHz bei einem Magnetfeld von 4 Gauss.

Vorzugsweise ist die zweite Einrichtung zum Erzeugen eines Wechselfeldes ausgebildet. Die beiden Einrichtungen zur Erzeugung des ersten und zweiten Magnetfeldes bilden hierbei insbesondere eine klassische Anordnung zur Erzeugung einer Kernspinresonanz. Dabei oszilliert das zweite Magnetfeld vorzugsweise mit der Spinresonanzfrequenz, welche im wesentlichen durch die Art der Teilchen, Elemente oder chemischen Verbindungen im Körper und durch die Stärke des ersten Magnetfeldes definiert wird. Die hervorgerufene Spinresonanzfrequenz beträgt bevorzugt zwischen 1 kHz und 1 MHz, besonders bevorzugt zwischen 2 kHz und 200 kHz und am meisten bevorzugt im Bereich von etwa 100 kHz.

Besonders vorteilhaft ist eine bevorzugte Ausführungsform, bei welcher die erste und zweite Einrichtung in einer Ebene angeordnet sind, welche Ebene parallel zu der Ebene der Matte verläuft. Dadurch lassen sich die erste und/oder zweite Einrichtung vorzugsweise vollständig innerhalb der Matte zwischen deren Ober- und Unterseite anordnen. Dadurch wird eine besonders einfache und praktische Bauform erzielt, wobei der Patient sich zur Behandlung einfach auf die Matte legt. Durch dieses Anordnung in einer Ebene wird außerdem eine plane Geometrie geschaffen, bei welcher dennoch im Behandlungsbereich zueinander orthogonale Magnetfelder erzeugt werden können.

Die Vorrichtung ist besonders leicht lager- und transportierbar, wenn gemäß einer bevorzugten Ausführungsform die Matte durch eine Unterteilung in mehrere Abschnitte ein- oder mehrfach faltbar ist. Hierbei sind die erste und zweite Einrichtung vorzugsweise in demselben Abschnitt der Matte untergebracht. Die Matte ist bevorzugt etwa 3 bis 10 cm stark, 70 cm breit und 210 cm lang, so dass in einem z.B. zweifach gefalteten Zustand Abmessungen von etwa 9 bis 30 cm mal 70 cm mal 70 cm erzielt werden.

Die zweite Einrichtung umfasst vorzugsweise eine Ringspule. Diese definiert eine Spulenebene, in welcher die Wicklungen verlaufen und eine zu der Spulenebene senkrechte Spulenachse. Im Zentrum der Spule wird, wie dem Fachmann offensichtlich ist, im wesentlichen ein Magnetfeld in Richtung der Spulenachse erzeugt. Die Spule oder zweite Einrichtung hat in Richtung der Spulenachse eine Ausdehnung von weniger als 50 cm, bevorzugt weniger als 20 cm, besonders bevorzugt weniger als 10 cm und am meisten bevorzugt zwischen etwa 2 cm und 6 cm. Die Spule oder zweite Einrichtung weist in der Spulenebene eine runde bis ovale oder längliche Form mit halbrunden Endbereichen auf. Insbesondere ist die Ausdehnung der Spule in Richtung der Spulenachse vorzugsweise kleiner, zumindest um einen Faktor 2 kleiner oder besonders bevorzugt zumindest um einen Faktor 5 kleiner als die Ausdehnung in der Spulenebene. Durch die besondere Formgebung wird insbesondere die vollständige Unterbringung in der flachen Matte bei gleichzeitig hoher Wirksamkeit des Magnetfeldes ermöglicht, was mit den bekannten großen Spulenanordnungen zumeist nicht möglich ist.

Die erste Einrichtung umfasst vorzugsweise zumindest eine, zwei, drei, besonders bevorzugt vier Spulen, wobei vorzugsweise jede dieser Spulen mit einem Festmagneten, z.B. einem Ferritmaterial kombiniert ist. Dadurch wird in vorteilhafter Weise ein starkes konstantes Basismagnetfeld, erzeugt durch das Ferritmaterial, und ein zeitlich veränderliches Zusatzmagnetfeld, erzeugt durch die Spulen, überlagert.

Somit kann mit relativ kleinen Spulen und geringem Energieverbrauch bei gleichzeitig effektivem Magnetfeld gearbeitet werden.

In einer bevorzugten Weiterbildung sind die erste und zweite Einrichtung zur Erzeugung des ersten bzw. zweiten Magnetfeldes in einer Ebene angeordnet, welche parallel zu der Ebene der Mattenoberfläche und der Spulenebene der zweiten Einrichtung verläuft. Umfasst die erste Einrichtung mehrere Spulen und/oder Festmagneten, wird die zweite Einrichtung bevorzugt zentral zwischen diesen angeordnet.

Insbesondere umfasst das Behandlungsfeld zumindest eine Überlagerung des ersten und zweiten Magnetfeldes. Im Behandlungsbereich oberhalb der Mattenoberfläche, insbesondere dort, wo sich ein Patient zur Behandlung befindet oder liegt, verlaufen die Magnetfeldlinien, welche von der ersten Einrichtung erzeugt werden, im wesentlichen parallel oder zumindest mit spitzem Winkel im Bereich von 0° bis 30° oder von 0° bis 45° zur Mattenoberfläche und/ oder senkrecht oder zumindest in stumpfen Winkel im Bereich von 45° oder 60° bis 120° oder 135° zu den Magnetfeldlinien der zweiten Einrichtung. Bevorzugt verläuft das zweite Magnetfeld in einem Winkel im Bereich von 30° bis 150°, besonders bevorzugt im Bereich von 45° bis 135°, besonders bevorzugt im Bereich von 60° bis 120° und am meisten bevorzugt im wesentlich senkrecht zur Mattenoberfläche.

In einer Ausführungsform der Erfindung ist das Behandlungsfeld derart zeitlich veränderbar, dass mittels der zeitlichen Veränderung des Behandlungsfeldes die Ausrichtung der Spins oder der durch die Spins erzeugten makroskopischen Magnetisierung in einer Weise veränderbar ist, dass eine Bewegung des im Behandlungsbereich angeordneten Körperbereichs im Erdmagnetfeld simulierbar ist.

Vorzugsweise umfasst das erste Magnetfeld eine im wesentlichen parallele oder antiparallele Überlagerung eines bevorzugt konstanten dritten Magnetfeldes, welches bevorzugt von den Festmagneten oder Ferriten erzeugt wird und eines bevorzugt zeitlich veränderlichen vierten Magnetfeldes, welches vorzugsweise von, den Festmagneten zugeordneten Hilfsspulen erzeugt wird. Hierbei beträgt die Stärke des dritten Magnetfeldes vorzugsweise 0,5 Gauss bis 500 Gauss, bevorzugt von 10 Gauss bis 50 Gauss und besonders bevorzugt im Bereich von 23 Gauss bis 24 Gauss. Das vierte Magnetfeld, welches auch als Modulationsfeld bezeichnet werden kann, oszilliert periodisch und vorzugsweise regelmäßig zwischen vorzugsweise -10 Gauss und +10 Gauss, bevorzugt zwischen -1 Gauss und +1 Gauss und besonders bevorzugt zwischen -0,5 Gauss und +0,5 Gauss, letzteres entspricht etwa der Stärke des Erdmagnetfeldes. Es ist dem Fachmann ersichtlich, dass das dritte Magnetfeld ein konstantes Basisfeld und das vierte Magnetfeld eine Amplitudenmodulation des ersten Magnetfeldes darstellen.

Vorzugsweise beschreibt das vierte Magnetfeld eine um 0 Gauss symmetrische dreiecks- oder sägezahnförmige Oszillation, so dass das erste Magnetfeld um den Wert des dritten Magnetfeldes oder konstanten Basisfeldes oszilliert. Folglich ist das erste Magnetfeld bevorzugt dreiecksförmig amplidutenmoduliert. Die mathematische Resonanzbedingung ist hierbei genau dann erfüllt, wenn das vierte Magnetfeld verschwindet. Die Stärke des dritten Magnetfeldes ist dabei zumindest 4 mal, 10 mal oder 20 mal so groß wie die maximale Stärke des vierten Magnetfeldes.

Wird nun das zweite Magnetfeld als Wechselfeld mit einer Frequenz, welche der Spinresonanzfrequenz der Teilchen im Gewebe in dem dritten Magnetfeld entspricht im wesentlichen senkrecht zu dem ersten Magnetfeld eingestrahlt, so entspricht dies einer Anordnung zur Herbeiführung eines sogenannten schnellen adiabatischen Durchlaufs.

Vorzugsweise weist das zweite Magnetfeld oder Wechselfeld während der ansteigenden und abfallenden Flanke des ersten Magnetfeldes verschiedene Intensitäten auf. Besonders bevorzugt wird das zweite Magnetfeld während der fallenden Flanke des ersten Magnetfeldes eingestrahlt und ist während der fallenden Flanke ausgeschaltet oder umgekehrt. Hierdurch werden die Spins oder die makroskopische Magnetisierung während der "An-Zeit" des zweiten Magnetfeldes adiabatisch aus der Richtung des Basisfeldes herausgedreht und relaxieren während der "Aus-Zeit" des zweiten Magnetfeldes wieder zurück.

Daher ist vorzugsweise die Frequenz des vierten Magnetfeldes oder der Amplitudenmodulation des ersten Magnetfeldes an die Spin-Gitter-Relaxationszeit der Teilchen im Gewebe angepaßt. Dies führt zu einer bevorzugten Periodendauer der Modulation des ersten Magnetfeldes von 1 ms bis 10 s, bevorzugt 10 ms bis 1 s und besonders bevorzugt im Bereich von 200 ms.

Alternativ zu der Anordnung für einen schnellen adiabatischen Durchlauf wird das zweite Magnetfeld oder Wechselfeld in einem kurzen Puls, z.B. einem sogenannten 90°-Puls oder einem 180°-Puls eingestrahlt.

Im folgenden wird die Erfindung anhand von bevorzugten Ausführungsformen und unter Bezugnahme auf die Zeichnungen näher erläutert.

Kurzbeschreibung der Figuren

Es zeigen:

Fig. 1a     eine Ansicht einer ersten Ausführungsform der Erfindung mit Bemaßung in mm,
Fig. 1b     eine Schnittzeichnung entlang der Schnittlinie A-A in Fig. 1a mit Bemaßung in mm,
Fig. 2     einen zeitlichen Verlauf eines Magnetfeldes B(t) und der resultierenden makroskopischen Magnetisierung M(t),
Fig. 3     eine Darstellung der Ausrichtung einer makroskopischen Magnetisierung M in einem konstanten Magnetfeld $B_0$,
Fig. 4     einen zeitlichen Verlauf eines Magnetfeldes B(t) und der resultierenden Magnetisierungskomponenten $M_z$

Fig. 5 einen Oszilloskopausdruck eines Kernspinresonanzsignals bei einer phasenempfindlichen Detektion mit 100 kHz Referenz,

Fig. 6 einen zeitlichen Ausschnitt eines Kernspinresonanzsignals bei $B_0$ = 23,4 Gauss,

Fig. 7 einen zeitlichen Ausschnitt eines Kernspinresonanzsignals bei $B_0$ = 23,2 Gauss,

Fig. 8 einen zeitlichen Ausschnitt eines Kernspinresonanzsignals bei $B_0$ = 23,8 Gauss,

Fig. 9 einen Verlauf der Magnetfeldstärke als Funktion der relativen Frequenz,

Fig. 10a eine schematische Darstellung der räumlichen Ausrichtung von Magnetfeldern beim schnellen adiabatischen Durchlauf zur Zeit $t_0$,

Fig. 10b wie Fig. 10a, zur Zeit $t_1$ statt $t_0$,

Fig. 10c wie Fig. 10a, zur Zeit $t_2$ statt $t_0$,

Fig. 11 eine schematische Darstellung des zeitlichen Verlaufs des ersten und zweiten Magnetfeldes und

Fig. 12 ein Blockschaltbild der erfindungsgemäßen Vorrichtung mit Steuerelektronik.

Fig 13a eine Ansicht einer zweiten Ausführungsform der Erfindung,

Fig. 13b eine Schnittzeichnung entlang der Schnittlinie A-A in Fig. 13a, und

Fig. 14 ein Blockschaltbild einer Schaltung zur Steuerung der Spulen der zweiten Ausführungsform.

<u>Detaillierte Beschreibung der Erfindung</u>

Durch Kernspinresonanz ist es möglich die Magnetisierungsrichtung im Körper zu ändern, ohne daß sich dabei der Körper'in Bewegung befindet, da die induzierte Kernspinresonanz-Spannung einen körpereigenen Bewegungsablauf simuliert. Man kann also mittels der erfindungsgemäßen Vorrichtung und des Verfahrens eine Therapie durchführen, welche den Stoffwechsel anregt bzw. beschleunigt.

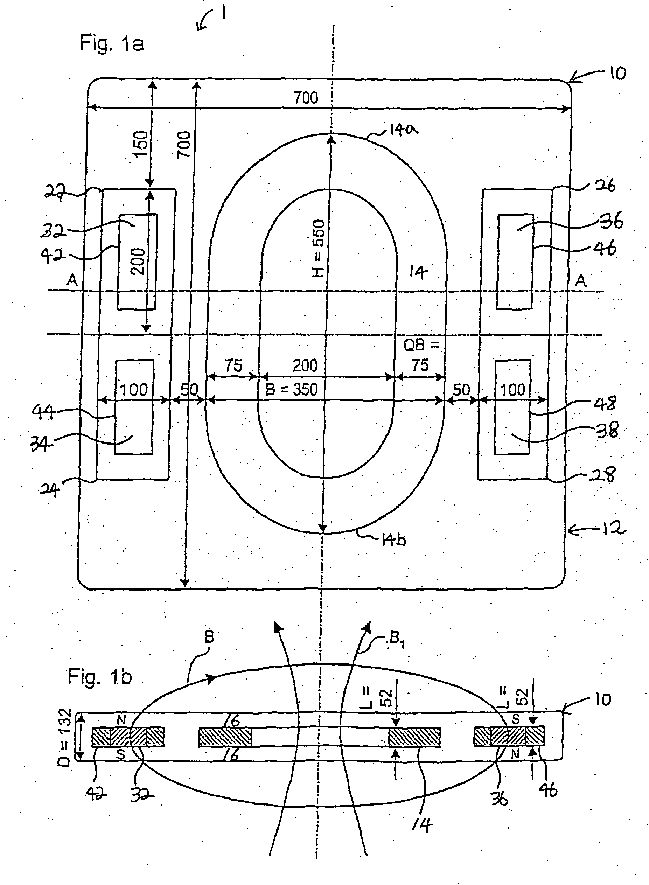

Figs. 1a und 1b zeigen eine erste Ausführungsform der Erfindung, wobei die dargestellten Maße nur exemplarisch zu verstehen sind. Die erfindungsgemäße Vorrichtung 1 umfasst eine in drei Abschnitte unterteilte und faltbare Matte 10, von welcher nur der mittlere Abschnitt 12 dargestellt ist und sich in der Zeichnungsebene erstreckt. In dem Abschnitt 12 der Matte 10 ist eine senkrecht zur Zeichnungsebene zweite Einrichtung in Form einer flachen Ringspule 14 zur Erzeugung eines zweiten Magnetfeldes in eine Polsterung 16 aus einem nachgiebigen Material, z.B. Schaumstoff eingebettet. Die Ringspule oder Sendespule 14 erstreckt sich in der Zeichnungsebene mit einer Breite von etwa B = 350 mm und einer Höhe von etwa H = 550 mm, wobei die Kopfenden 14a, 14b jeweils halbrund ausgebildet sind. Die Länge der Spule senkrecht zur Zeichnungsebene beträgt etwa L = 52 mm. Der Querschnitt der Spulen wird definiert durch die Länge L und eine Querschnittsbreite von etwa QB = 75 mm. Die Dicke der Matte beträgt etwa D = 132 mm, wobei die Ringspule zentriert in der Matte angeordnet ist, so dass zwischen der Mattenober- und -unterseite jeweils noch etwa 40 mm Polsterung 16 angeordnet sind. Links und rechts der Spule befinden sich je zwei Einrichtungen 22, 24, 26, 28 zur Erzeugung eines ersten Magnetfeldes, welche je einen Festmagneten 32, 34, 36, 38 und je eine Hilfsspule 42, 44, 46, 48 umfassen welche in der Mattenebene die Festmagnete umschließen. Jede Einrichtung 22, 24, 26, 28 hat eine Höhe von etwa 200 mm, eine Breite von etwa 100 mm und eine Länge L senkrecht zur Zeichenebene von etwa 52 mm. Die Einrichtungen 22, 24, 26, 28 sind jeweils in Richtung der Breite etwa 50 mm von der Ringspule 14 beabstandet und je zwei der Einrichtungen 22 und 24 sowie 26 und 28 grenzen in Richtung der Höhe aneinander an.

<u>90°-Kernspinresonanz-Signal-Impulsverfahren</u>

Wie vorstehend bereits ausgeführt ist, verwendet eine erste Ausführungsform der Erfindung ein Impulsverfahren, welches im Folgenden detailliert beschrieben ist.

Die Moleküle oder Makromolekülkomplexe unseres Körpers beinhalten zum Großteil Wasserstoffatome, z.B. in Wasser ($H_2O$) oder in organischen Molekülen (z.B. in $CH_2$ oder $CH_3$). Die Kerne oder Ionen des Wasserstoffs sind Protonen. Protonen haben ein magnetisches Moment und einen Spin (anschaulich ein Drehmoment), welche im Verhältnis $\gamma$ (gyromagnetischer Faktor) zueinander stehen. Für Protonen ist $\gamma$ = 2,67522 · $10^8$ $T^{-1}s^{-1}$.

Ein statisches Magnetfeld $B_0$, z.B. das Erdmagnetfeld, erzeugt exponentiell in der Zeit mit einer Zeitkonstante $T_1$ eine makroskopische Magnetisierung M(t). Hierfür gilt

$$M(t) = M_0(1-e^{-t/T1})$$

mit

$$M_0 = \chi B_0$$

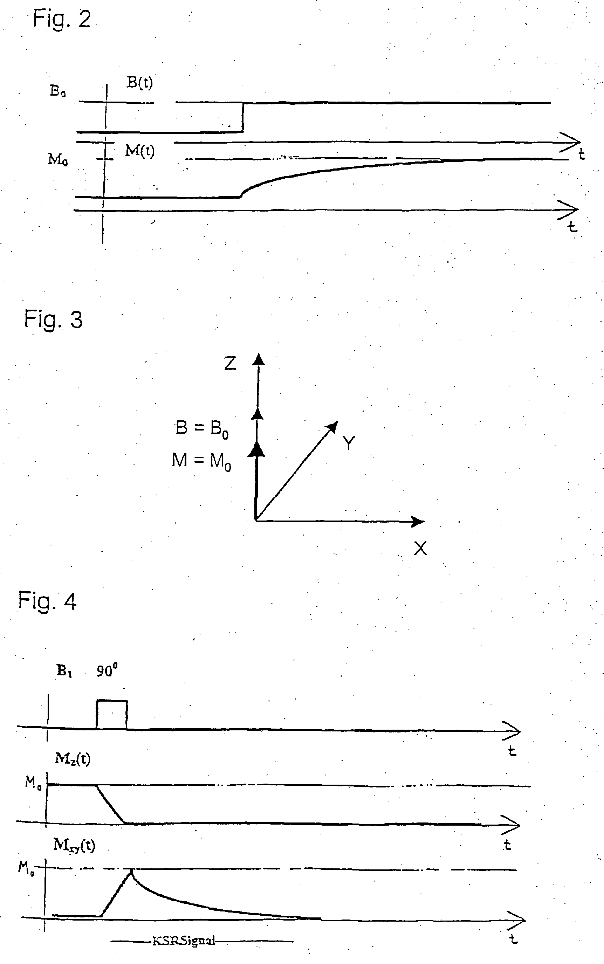

wobei $T_1$ die Spin-Gitter-Relaxationszeit und $M_0$ der asymptotische Wert der Magnetisierung ist. Der zeitliche Verlauf der Magnetisierung M(t), welcher durch sprunghaftes Anlegen eines nach dem Anstieg konstanten Magnetfeldes $B_0$ erzeugt wird, ist in Fig. 2 veranschaulicht. Für Protonen oder Wasserstoff im menschlichen Gewebe gilt:

$$T_1 = 10s \ ... \ 10^{-3} \ s.$$

Vorzugsweise wird eine Spin-Echo-Messung vor der therapeutischen Behandlung durchgeführt, um die Spin-Gitter-Relaxationszeit zu bestimmen.

Asymptotisch richtet sich die makroskopische Magnetisierung M parallel zu dem angelegten Magnetfeld B = $B_0$ aus, wie in Fig. 3 veranschaulicht ist. Fig. 3 zeigt außerdem ein rechtwinkliges und rechtshändiges Koordinatensystem XYZ, welches für die folgende Betrachtungsweise als Orientierung zugrunde gelegt wird.

Mikroskopisch und quantenmechanisch gefordert üben alle Protonenspins eine Präzesionsbewegung um $B_0$ mit einer Frequenz $f_0$ aus. Diese Frequenz wird als Larmorfrequenz bezeichnet. Die Larmorfrequenz $f_0$ bestimmt sich wie folgt

$$f_0 = \frac{\omega_0}{2\pi}.$$

Daraus ergibt sich im Erdmagnetfeld, d.h. für $B_0 = 0{,}5$ Gauss = $5 \cdot 10^{-5}$ T

$$f_0 = \frac{\gamma B_0}{2\pi} = \frac{2{,}67522 \cdot 10^8 \cdot 5 \cdot 10^{-5}}{2\pi} \, Hz = 2128{,}872 \, Hz$$

Im Erdmagnetfeld beträgt die Larmorfrequenz von Protonen folglich etwa 2 kHz. Die Larmorfrequenz wird auch durch die chemischen Bindungen nur sehr geringfügig verändert.

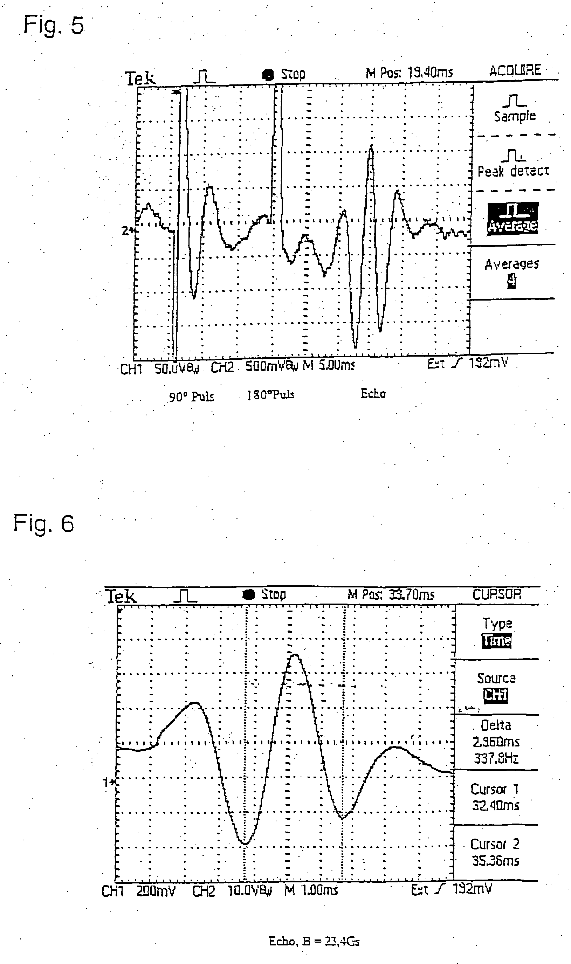

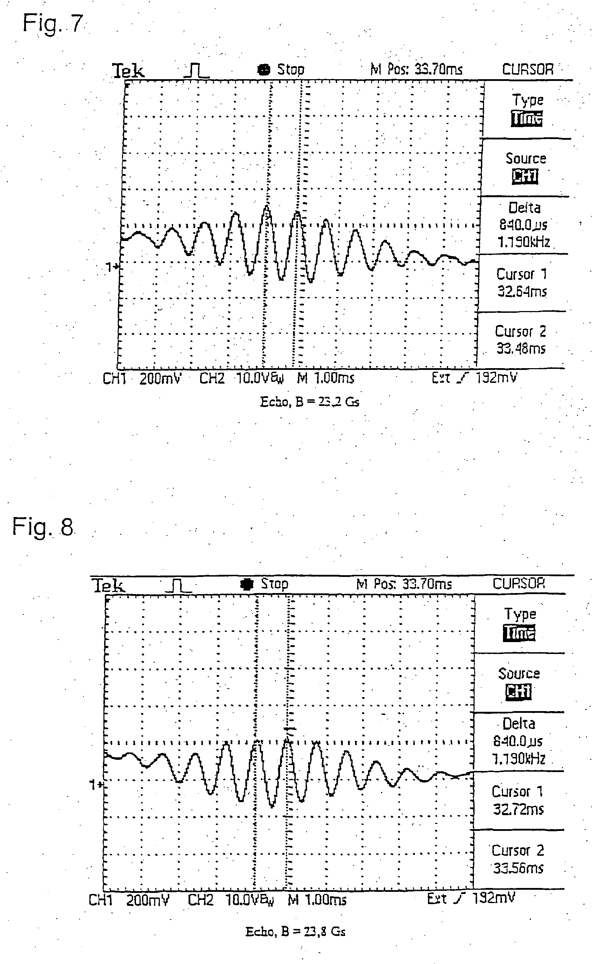

Fig. 5 zeigt einen Oszilloskopausdruck zum experimentellen Nachweis der Larmorfrequenz mittels einer Spin-Echo-Messung an 500 ml Wasser mit einem 100 kHz und einem 23,5 Gauss Spektrometer. Es werden ein 90°-Puls und ein 180°-Puls eingestrahlt und das Spin-Echo detektiert. Die Figs. 6 bis 8 zeigen die Spinechos bei einem ersten Magnetfeld von B = 23,2 Gauss, 23,4 Gauss bzw. 23,8 Gauss mit vergrößerter Zeitskala. Das erste Magnetfeld B wird durch parallele Überlagerung eines konstanten Magnetfeldes $B_0$, erzeugt durch die vier Festmagnete, welche als FerritMagnete 32, 34, 36, 38 ausgeführt sind und eines zeitlich veränderlichen Magnetfeldes $\Delta B_0$, erzeugt durch die vier Hilfsspulen 42, 44, 46, 48, hergestellt.

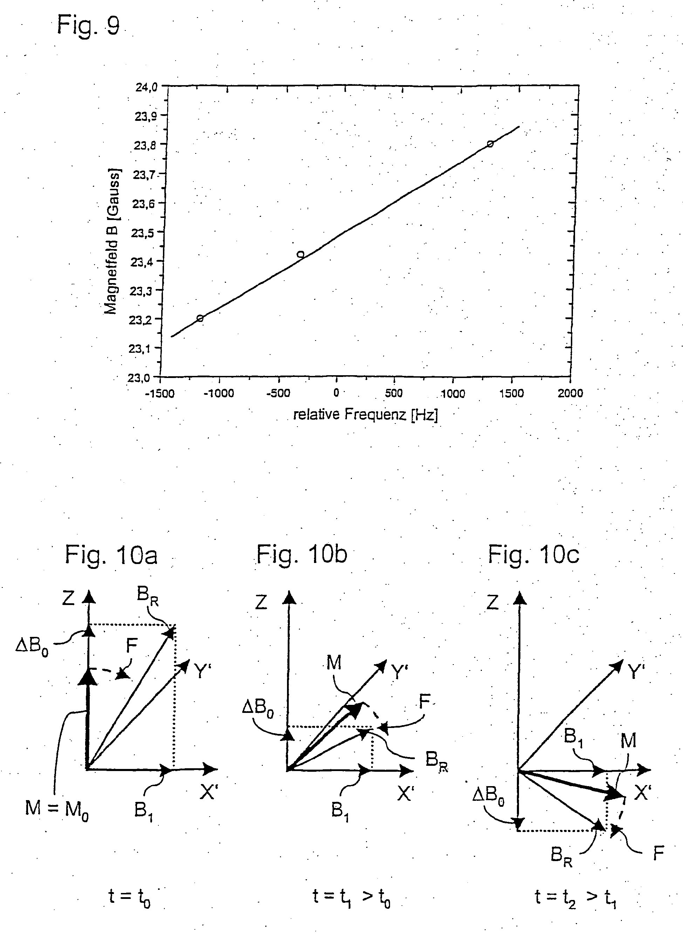

In Fig. 9 sind die drei Messpunkte der Figs. 6 bis 8 in einem Graph des Magnetfeldes in Gauss als Funktion der relativen Frequenz in Hz dargestellt. Die Gerade ist eine lineare Interpolation durch die Messpunkte. Die relative Frequenz repräsentiert die Frequenzabweichung von der Resonanzfrequenz $f_0$, welche durch das Basisfeld $B_0 = 23{,}5$ Gauss definiert wird.

Die erfindungsgemäße Vorrichtung umfasst die flache Spule oder Sendespule 14 zur Erzeugung des zweiten Magnetfeldes in Form eines magnetischen Wechselfeldes $B_1$ mit einer Frequenz $f_0$ von etwa 100 kHz. Diese Frequenz entspricht etwa der Larmorfrequenz von Protonen in einem mittleren Magnetfeld von B = 23,5 Gauss.

Hierzu wird die Sendespule 14 vorzugsweise und in sehr einfacher Weise mit einem Kondensator zu einem Schwingkreis geschaltet. Die Resonanzfrequenz des Schwingkreises $f_{LC}$ ist

$$f_{LC} = \frac{1}{2\pi\sqrt{LC}},$$

wobei L die Induktivität der Sendespule 14 und C die Kapazität des Kondensators sind.

Befinden sich Körperbereiche eines Patienten oder biologisches Gewebe in dem ersten Magnetfeld B, welches zunächst eine konstante Stärke $B_0 = 23,5$ Gauss aufweist, richtet sich die makroskopische Magnetisierung M des Gewebes als Vektorsumme der Kernspins parallel zu $B_0$ aus, wobei $B_0$ in dieser Ausführungsform parallel zur Z-Achse verläuft (siehe Fig. 3).

Um die Magnetisierung M nun aus der $B_0$-Richtung abzulenken, wird ein Kernspinresonanz-Verfahren eingesetzt. Durch Kernspinresonanz wird die Magnetisierungsrichtung geändert, obwohl der Körper sich in Ruhe befindet. Die induzierte Spannung wirkt, als ob der Körper in Bewegung wäre. Man kann so durch Kernspinresonanz eine Therapie durchführen, die den Stoffwechsel anregt.

Mit einem sogenannten 90°-Radio-Frequenz-Impuls wird die Magnetisierung um 90° Grad gedreht. Der zeitliche Verlauf des Magnetfeldes und der Magnetisierungskomponenten $M_z(t)$ und $M_{xy}(t)$ ist schematisch in Fig. 4 dargestellt. Die Sendespule, deren Achse parallel zur X-Achse verläuft, generiert ein rotierendes oder in X-Richtung linear schwingendes Hochfrequenz-Feld $B_1$. Die makroskopische Magnetisierung M dreht sich mit einer Frequenz $f_1$ um die X-Achse von der positiven Z-Richtung bis in die X-Y-Ebene. Hierbei gilt

$$f_1 = \omega_1 / 2\pi$$

mit

$$\omega_1 = \gamma B_1$$

Der Winkel $\alpha$, um welchen sich M dreht, beträgt

$$\alpha = \omega_1 t.$$

Für eine 90°-Drehung, d.h. $\alpha = \pi/2$, errechnet sich die zeitliche Länge des 90°-Pulses $t_{90}$ als

$$t_{90} = \frac{\pi}{2} \frac{1}{\gamma B_1} .$$

Die makroskopische Magnetisierung M steht nach Einstrahlung des 90°-Pulses in Richtung Y. Sie dreht sich mit $\omega_0$ um die Z-Achse und induziert eine Spannung in der Hochfrequenz-Spule, welche als Kernspinresonanz-Signal messbar ist. Dieses Signal klingt exponentiell mit der Zeitkonstanten $T_2^*$ ab. Es gilt

$$M_{xy} = M_0 \cdot e^{-t/T2^*}$$

Für ein homogenes Magnetfeld B gilt: $T_2^* \cong T_2$, wobei $T_2$ die Spin-Spin-Relaxationszeit ist.
Für ein weniger homogenes Magnetfeld B gilt: $T_2^* < T_2$
Für Flüssigkeiten gilt: $T_1 \cong T_2$
Typische Werte für $T_2$ sind:

Leitungswasser: $T_2 \cong 3$ s

Destilliertes Wasser: $T_2 \cong 30$ s bis 3 min

Menschliches Gewebe: $T_2 \cong 10$ ms bis 1 s

Gewebe einer Hand: $T_2 \cong 100$ ms bis 1 s.

Schneller adiabtischer Kernspinresonanzdurchlauf

Bezugnehmend auf Figs. 10a bis 10c und alternativ zu dem vorstehend beschriebenen Impulsverfahren wird eine gezielte Drehung der Magnetisierung durch einen schnellen adiabatischen Durchlauf erreicht, welcher im folgenden beschrieben wird. Dieser wird durch eine Feldänderung des ersten Magnetfeldes B oder eine Frequenzänderung des Wechselfeldes $B_1$ erzielt, wobei die Magnetisierung M von 0 bis 180° bezüglich der Z-Achse rotiert werden kann.

In einem um die Z-Achse mit $\omega_0$ rotierenden Koordinatensystem (X' Y' Z), sind folgende Magnetfelder definiert:

$$\Delta B_0 = B - B_0 \quad mit \quad B_0 = \omega_0 / \gamma$$

$$B_1 \quad und$$

$$B_R.$$

Hierbei ist $B_1$ ein Wechselfeld oder Hochfrequenz-Feld, welches durch die Sendespule 14 erzeugt wird und zum Zeitpunkt $t = t_0$ im Koordinatensystem X'Y'Z parallel zur X'-Achse verläuft. $B_R$ ist das aus einer Überlagerung von B und $B_1$ resultierende Magnetfeld oder Behandlungsfeld.

Fig. 10a zeigt die Ausrichtung der Magnetfeldvektoren im Raum in einer Momentaufnahme zur Zeit $t_0$. Dargestellt sind der Vektor des vierten Magnetfeldes $\Delta B_0$, erzeugt durch die Hilfsspulen 42, 44, 46, 48 welcher parallel zur Z-Achse verläuft. Dabei ist $\Delta B_0$ der positive oder negative Überschuss des Magnetfeldes B über bzw. unter das resonante dritte Magnetfeld $B_0$, welches durch die Ferritmagnete 32, 44, 46, 48 erzeugt wird, dauerhaft in positive Z-Richtung zeigt und nicht in den Figs. 10a bis 10c dargestellt ist.

Wirkt zunächst nur das Magnetfeld $B(t) = B_0$, so richtet sich die makroskopische Magnetisierung des Gewebes in Richtung der Z-Achse aus und die einzelnen Spins präzedieren mit der Winkelfrequnz $\omega_0$ um die Z-Achse. D.h. bezüglich des rotierenden Koordinatensystems X'Y'Z ruhen die Spins zunächst.

Nun werden das dritte Magnetfeld $\Delta B_0$ und das Wechselfeld $B_1$, welche sich zum resultierenden Magnetfeld $B_R$ überlagern, bis zum Zeitpunkt $t = t_0$ hochgefahren. Der Vektor des Wechselfeldes $B_1(t_0)$ zeigt in Richtung der X'-Achse.

Das Wechselfeld $B_1$ schwingt linear mit der Frequenz $\omega_0$ im wesentlichen senkrecht zur Z-Achse. Alternativ kann das Feld $B_1$ auch mit der Frequenz $\omega_0$ um die Z-Achse rotieren. Bezüglich der Projektion in die X'-Z-Ebene ist dies äquivalent. Da auch die Kernspins mit derselben Frequenz $\omega_0$ um die Z-Achse rotieren, liegen diese immer in Phase mit dem Wechselfeld $B_1$.

Gemäß einer klassischen Interpretation greift bei dieser Anordnung dauerhaft eine resultierende Kraft F an der Magnetisierung oder den Spins an, wobei diese Kraft F die Magnetisierung M oder die Spins in der X'-Z-Ebene von der Z-Achse wegdreht. Die Spins präzedieren während dieser Drehung im wesentlichen in Phase. Mit dieser Drehung wird das vierte Magnetfeld oder Modulationsfeld $\Delta B_0$ bis auf null verringert und weiter kontinierlich in negativer Z-Richtung wieder erhöht, um der Änderung Magnetisierungsrichtung zu folgen. Es kann so eine Drehung der Magnetisierung bis in Richtung der negativen Z-Achse, also eine Drehung der Magnetisierung der Kerne um 180° erreicht werden.

Fig. 10b zeigt die Ausrichtung der Magnetisierung M und der verschiedenen magnetischen Felder zu einer Zeit $t_1$, welche später liegt als $t_0$. Der Magnetisierungsvektor M ist bereits deutlich von der Z-Achse weggedreht.

Entsprechend zeigt Fig. 10c eine Momentaufnahme zu einer Zeit $t_2$ welche noch später als $t_1$ liegt.

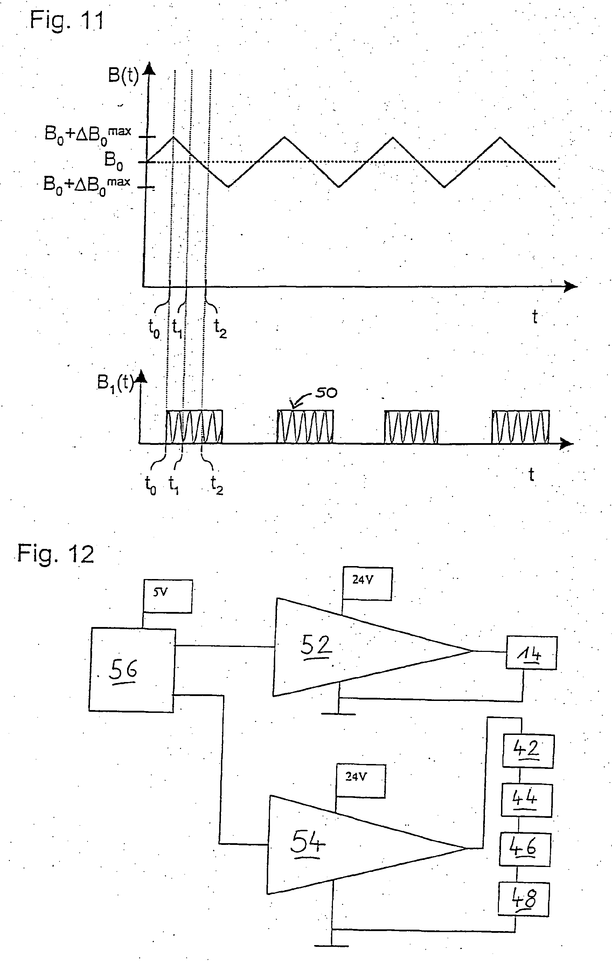

Um den erwünschten Effekt einer Bewegungssimulation zu maximieren, soll die Magnetisierung M möglichst häufig gedreht werden. Hierfür wird die Hilfsspule, welche das Magnetfeld $\Delta B_0$ erzeugt, dreiecksförmig, sägezahnartig oder sinusförmig zwischen $\Delta B_0^{max}$ und $-\Delta B_0^{max}$, also symmetrisch um null gefahren. Fig. 11 oben zeigt schematisch die am meisten bevorzugte dreiecksförmige Modulation des ersten Magnetfeldes B(t). Weiter sind die Zeiten $t_0$, $t_1$ und $t_2$ aus Figs. 10a bis 10c eingezeichnet. Bei fallendem Magnetfeld B(t) ist die Sendespule bzw. das Wechselfeld $B_1$ eingeschaltet, um die Magnetisierung von der positiven Z-Achse weg zu drehen, während die Sendespule bei ansteigendem Feld ausgeschaltet ist. Folglich ist das Wechselfeld $B_1(t)$ gemäß diesem Ausführungsbeispiel rechteckförmig amplitudenmoduliert. Andere Modulationsformen für das erste und/oder zweite Magnetfeld, z.B. eine sinusförmige Amplitudenmodulation liegen aber ebenfalls im Rahmen der Erfindung. Die in Fig. 11 unten dargestellten Blöcke 50 repräsentieren schematisch die An-Zeit des Wechselfeldes $B_1$. Während der Aus-Zeit des Wechselfeldes $B_1$ relaxieren die Spins bzw. die Magnetisierung wieder zurück. Daher ist die Modulationsperiode des ersten bzw. vierten Magnetfeldes an die Spin-Gitter-Relaxationszeit des Gewebes angepasst oder entspricht dieser zumindest größenordnungsmäßig. So beträgt die Periode der zeitlichen Veränderung des ersten Magnetfeldes vorzugsweise ein Zehntel bis 10 mal, insbesondere ein mal bis 3 mal oder 5 mal die Spin-Gitter-Relaxationszeit.

Es liegt auch im Rahmen der Erfindung die fallende Flanke des Modulationsfeldes $\Delta B_0$ steiler anzulegen als

die ansteigende, um eine schnellere Drehung zu erreichen.

Vorstehend wurde der adiabatische Durchlauf mittels einer Modulation des ersten Magnetfeldes B(t) erläutert. Der Durchlauf läßt sich analog auch mit einem konstanten ersten Magnetfeld B = $B_0$ und einer entsprechenden Frequenzänderung (sogenannter Frequenzsweep) des Wechselfeldes $B_1$ durchführen.

Zusätzlich detektiert eine Empfangsspule mit der Achse in Y-Richtung das induzierte Kernspinresonanz-Signal phasenempfindlich. Das zeitliche Integral dieses Signals ist proportional zur gesamten Kernspinresonanz-Wirkung und ist daher maximiert.

Ein Vorteil des adiabatischen Durchlaufs ist es, dass das erste Magnetfeld B bis zu etwa 10 % Inhomogenität aufweisen kann. D.h. das Verfahren ist diesbezüglich um Größenordnungen unempfindlicher als bekannte Verfahren, wie z.B. Spin-Echo-Verfahren. Entsprechend unempfindlich ist die Erfindung auch bezüglich des Winkels zwischen dem ersten und zweiten Magnetfeld.

Fig. 12 zeigt eine beispielhafte Schaltungsanordnung der erfindungsgemäßen Vorrichtung mit je einem Verstärker 52 und 54 zum Treiben der Sendespule 14 bzw. der Hilfsspulen 42, 44, 46, 48. Eine Steuereinrichtung oder -logik 56 ist der Sendespule 14 und den Hilfsspulen 42, 44, 46, 48 bzw. den beiden Verstärkern 52 und 54 zugeordnet und steuert die Modulation des ersten und zweiten Magnetfeldes.

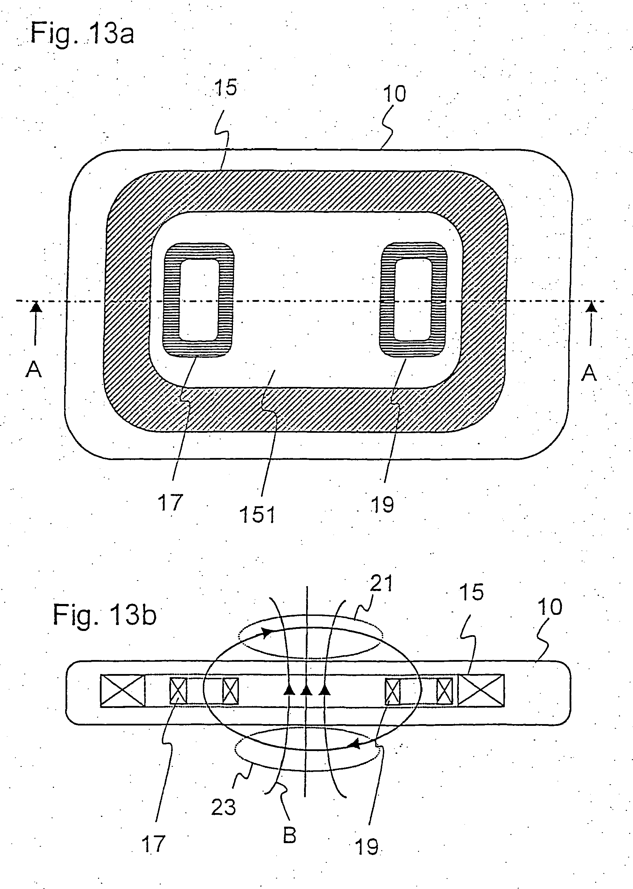

Die Figuren 13a und 13b zeigen eine zweite Ausführungsform der Erfindung. Dabei zeigt Fig. 13a eine Ansicht dieser zweiten Ausführungsform und Fig. 13b eine Schnittzeichnung entlang der Schnittlinie A-A in Fig. 13a. Die Matte 10 weist eine flache Ringspule 15 auf, in deren Innenbereich 151 zwei weitere flache Ringspulen 17 und 19 angeordnet sind. Ebenso wie die anhand der Figuren 1a und 1b beschriebene Ausführungsform ist auch diese Ausführungsform geeignet, beispielsweise das 90°-Kernspinresonanz-Signal-Impulsverfahren und den schnellen adiabatischen Kernspinresonanzdurchlauf zu implementieren.

Mit der Ringspule 15 wird ein quasistatisches Magnetfeld B(t)=$B_0$+$\Delta B_0$(t) erzeugt. Um einen großen Behandlungsbereich zu erzielen, ist $\Delta B_0$(t) betragsmäßig bevorzugt etwa halb so groß wie $B_0$.

Die flachen Ringspulen 17 und 19 werden gegenläufig betrieben, so daß jeweils ein Nord- und ein Südpol der beiden Spulen zu einer Seite der Matte 10 zeigt. Auf diese Weise wird von diesen Spulen ein Magnetfeld $B_1$ erzeugt, welches in Bereichen 21 und 23 oberhalb und unterhalb der Matte 10 im wesentlichen senkrecht zu dem von der Ringspule 15 erzeugten Magnetfeld B steht. Liegt ein Patient auf der Matte, so befindet sich Gewebe des Patienten innerhalb dieses Bereiches 21. Der Bereich 21 definiert somit einen Behandlungsbereich für das zu therapierende Gewebe.

Der zeitliche Verlauf des Magnetfelds B(t), sowie des Magnetfelds $B_1$ wird dabei wie oben anhand der weiteren Ausführungsformen beschrieben wurde, gesteuert.

Im Unterschied zur ersten Ausführungsform der Erfindung verläuft das Magnetfeld B im Behandlungsbereich jedoch eher senkrecht zur Mattenoberfläche, beziehungsweise senkrecht zu dem von den Spulen 22, 24, 26 und 28 erzeugten Magnetfeld B(t) der ersten Ausführungsform. Weiterhin werden bei der zweiten Ausführungsform keine Festmagneten benötigt. Die konstante Magnetfeldkomponente $B_0$ kann vielmehr ebenso wie das zeitlich veränderliche Magnetfeld $\Delta B_0$, durch geeignete Ansteuerung der Ringspule 15 erzeugt werden.

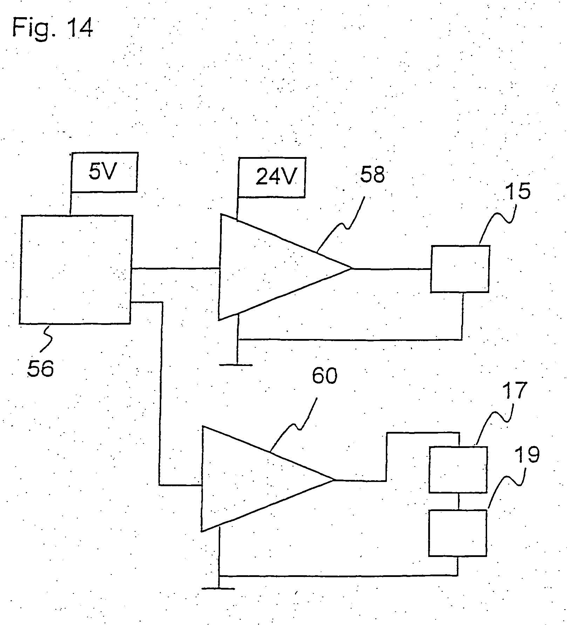

Eine geeignete Steuerung der Spulen 15, 17 und 19 zur Erzeugung eines quasistatischen Magnetfelds B(t), sowie einem Wechselfeld $B_1$(t) mit einem beispielsweise wie in Fig. 11 dargestellten zeitlichen Verlauf ist in Fig. 14 anhand eines Blockschaltbilds dargestellt. Die Steuerung weist, ähnlich wie die anhand von Fig. 12 dargestellte Steuerung eine Logikschaltung 56 auf. Mit der Logikschaltung 56 werden ein Verstärker 58 zum Treiben der Ringspule 15, sowie ein Verstärker zum Treiben der Ringspulen 17 und 19 zur Erzeugung des Wechselfelds $B_1$ angesteuert. Der Verstärker 58 erzeugt dabei für eine Ausführungsform ohne Permanentmagnete einen Konstantstrom, welcher in der Spule 15 ein konstantes Magnetfeld $B_0$ bewirkt, sowie einen darauf aufgesetzten, zeitlich veränderlichen Strom, welcher die veränderliche Magnetfeldkomponente $\Delta B_0$ bewirkt.

Zusammenfassend wird festgehalten, dass die vorliegende Erfindung eine Magnetfeld-Therapievorrichtung und ein Magnetfeld-Therapieverfahren vorschlägt, welche das Kernspinresonanz-Signal als Bewegungsfühler verwenden, um den Stoffwechsel zu stimulieren. Das Signal simuliert dabei die Bewegung eines Körperteils. Vorteilhaft ist hierbei, dass die vorgeschlagene Kernspinresonanztherapie aller Wahrscheinlichkeit nach keine negative Wirkungen auf den Organismus ausübt.

Mittels der erfindungsgemäßen Kernspinresonanz-Therapievorrichtung, kann die Magnetisierung mit geringer Energie und schnell gedreht werden. Die Drehung erfolgt insbesondere innerhalb einer Mikrosekunde bis zu 30 Sekunden.

**Patentansprüche**

**1.** Vorrichtung zur Behandlung mit magnetischen Feldern für die Behandlung von Tieren umfassend:

eine erste Einrichtung zur Erzeugung eines ersten Magnetfeldes,
eine zweite Einrichtung zur Erzeugung eines zweiten Magnetfeldes und
einen Träger, insbesondere eine Matte mit einer Oberseite und einer Unterseite, wobei die Matte zum Auflegen von zu behandelnden Körperbereichen eines Patienten ausgebildet ist.

**2.** Vorrichtung nach Anspruch 1, wobei
die zweite Einrichtung zum Erzeugen eines Wechselfeldes ausgebildet ist.

**3.** Vorrichtung nach Anspruch 1 oder 2, wobei
in einem Behandlungsbereich oberhalb der Oberseite des Trägers, insbesondere der Matte das erste Magnetfeld in spitzen Winkel und/oder das zweite Magnetfeld in stumpfem Winkel zu der Oberseite verläuft.

**4.** Vorrichtung nach Anspruch 1, 2 oder 3, wobei

Elemente und/oder Verbindungen, welche in den zu behandelnden Körperbereichen enthalten sind, in dem ersten Magnetfeld zumindest eine Spinresonanzfrequenz aufweisen und
das zweite Magnetfeld zumindest zeitweise mit einer Frequenz oszilliert, welche Frequenz der Spinresonanzfrequenz entspricht.

**5.** Vorrichtung nach einem der vorstehenden Ansprüche, wobei
die erste und zweite Einrichtung in einer ersten Ebene angeordnet sind, die Oberseite des Trägers, insbesondere der Matte sich in einem Funktionszustand im wesentlichen in einer zweiten Ebene erstreckt und die erste Ebene im wesentlichen parallel zu der zweiten Ebene verläuft.

**6.** Vorrichtung nach einem der vorstehenden Ansprüche, wobei
die zweite Einrichtung eine Spule umfasst.

**7.** Vorrichtung nach einem der vorstehenden Ansprüche, wobei
die zweite Einrichtung eine Ringspule umfasst, deren Spulenachse im wesentlichen senkrecht zur Oberfläche des Trägers, insbesondere der Matte angeordnet ist.

**8.** Vorrichtung nach einem der vorstehenden Ansprüche, wobei
die erste Einrichtung zwei oder vier Spulen umfasst, zwischen welchen die zweite Einrichtung angeordnet ist.

**9.** Vorrichtung nach einem der vorstehenden Ansprüche, wobei
der Träger, insbesondere die Matte eine Füllung umfasst, in welche die erste und/oder zweite Einrichtung eingebettet sind.

**10.** Vorrichtung nach einem der vorstehenden Ansprüche, umfassend eine, vorzugsweise gepolsterte, Hülle mit einer verschließbaren Öffnung zum Einbringen und/oder Entfernen der ersten und/oder zweiten Einrichtung.

**11.** Vorrichtung nach einem der vorstehenden Ansprüche, wobei
die Matte in zwei, drei oder eine Vielzahl von Abschnitten untergliedert ist und der Träger, insbesondere die Matte zwischen den Abschnitten faltbar ist.

**12.** Vorrichtung nach einem der vorstehenden Ansprüche, wobei
die erste Einrichtung zum Erzeugen eines zeitlich veränderlichen Magnetfeldes ausgebildet ist.

**13.** Vorrichtung nach einem der vorstehenden Ansprüche, wobei
die erste Einrichtung zumindest einen Festmagneten und zumindest eine Spule umfasst.

**14.** Vorrichtung nach einem der vorstehenden Ansprüche, wobei
Intensität und/oder Richtung des ersten Magnetfeldes veränderbar sind.

**15.** Vorrichtung nach einem der Ansprüche 1 bis 14, **dadurch gekennzeichnet, daß** der Träger eine Behandlungsliege und/oder einen Behandlungsstuhl und/oder eine mehrflügelige Anordnung, welche sich um einen Körperteil, insbesondere den Kopf eines Patienten legen läßt, und/oder eine Gamasche und/oder eine Decke umfaßt.

**16.** Vorrichtung zur therapeutischen Behandlung mit magnetischen Feldern für die Behandlung von Tieren, insbesondere für die Vorrichtung nach einem der vorstehenden Ansprüche, definierend einen Behandlungsbereich, in dem Gewebe zumindest eines Körperbereichs eines zu behandelnden Patienten anordenbar ist, die Vorrichtung umfassend:

eine erste Einrichtung zur Erzeugung eines ersten Magnetfeldes in dem Behandlungsbereich
eine zweite Einrichtung zur Erzeugung eines zweiten Magnetfeldes, welches sich in dem Behandlungsbereich mit dem ersten Magnetfeld zu einem Behandlungsfeld überlagert, wobei die in dem Gewebe enthaltenen Elemente und/oder Verbindungen in dem Behandlungsfeld zumindest eine Spinresonanzfrequenz aufweisen und das zweite Magnetfeld ein magnetisches Wechselfeld umfasst, welches zumindest zeitweise eine Frequenz aufweist, welche der Spinresonanzfrequenz entspricht und
eine Steuereinrichtung zur Steuerung zumindest einer der beiden ersten und zweiten Einrichtungen, mit welcher Steuereinrichtung das Behandlungsfeld zeitlich veränderbar ist, wobei mittels der zeitlichen Veränderung des Behandlungsfeldes die Ausrichtung der Spins veränderbar ist.

**17.** Vorrichtung nach Anspruch 16, wobei
die Ausrichtung einer makroskopischen Magnetisierung, welche durch die Spins erzeugt ist, mittels der zeitlichen Veränderung des Behandlungsfeldes veränderbar ist.

**18.** Vorrichtung nach Anspruch 16 oder 17, umfassend Mittel, insbesondere eine Steuereinrichtung für das Behandlungsfeld zur adiabatischen Veränderung der Richtung der Magnetisierung des Gewebes.

**19.** Vorrichtung nach einem der Ansprüche 16 bis 18, wobei das zweite Magnetfeld im wesentlichen senkrecht zu dem ersten Magnetfeld verläuft.

**20.** Vorrichtung nach einem der Ansprüche 16 bis 19, wobei das zweite Magnetfeld im wesentlichen räumlich linear und/oder senkrecht zu dem ersten Magnetfeld oszilliert.

**21.** Vorrichtung nach einem der Ansprüche 16 bis 20, wobei das erste Magnetfeld im Behandlungsbereich eine Stärke von 0,5 Gauss bis 500 Gauss, insbesondere von 10 Gauss bis 50 Gauss, insbesondere im Bereich von 23 Gauss bis 24 Gauss aufweist.

**22.** Vorrichtung nach einem der Ansprüche 16 bis 21, wobei das zweite Magnetfeld im Behandlungsbereich eine Frequenz von 1 kHz bis 1 Mhz, insbesondere von 2 kHz bis 200 kHz und insbesondere im Bereich von 100 kHz aufweist.

**23.** Vorrichtung nach einem der Ansprüche 16 bis 22, wobei das zweite Magnetfeld amplitudenmoduliert ist.

**24.** Vorrichtung nach einem der Ansprüche 16 bis 23, umfassend Mittel zur zeitlichen Veränderung der Intensität und/oder der Richtung des ersten Magnetfelds.

**25.** Vorrichtung nach einem der Ansprüche 16 bis 24, wobei das erste Magnetfeld amplitudenmoduliert ist und insbesondere die Periodendauer der Amplitudenmodulation an die Spin-Gitter-Relaxationszeit des zu behandelnden Gewebes angepasst ist.

**26.** Vorrichtung nach Anspruch 25, wobei
das erste Magnetfeld zwischen Minimum und Maximum der Magnetfeldfeldstärke einen Wert annimmt, bei welchem die Frequenz des zweiten Magnetfeldes der Spinresonanz-frequenz entspricht.

**27.** Vorrichtung nach Anspruch 25 oder 26, wobei
die Periodendauer der Veränderung des ersten Magnetfeldes 1 ms bis 10 s, insbesondere 10 ms bis 1 s, insbesondere im Bereich von 200 ms beträgt.

**28.** Vorrichtung nach einem der Ansprüche 25 bis 27, wobei das zweite Magnetfeld während fallendem ersten Magnetfeld eingeschaltet und während ansteigendem ersten Magnetfeld abgeschaltet ist oder umgekehrt.

**29.** Vorrichtung nach einem der Ansprüche 16 bis 28, wobei die erste Einrichtung eine dritte Einrichtung mit einem Festmagneten zur Erzeugung eines statischen dritten Magnetfeldes und eine vierte Einrichtung zur Erzeugung eines zeitlich veränderlichen vierten Magnetfeldes umfasst, wobei das erste Magnetfeld eine Überlagerung des dritten und vierten Magnetfeldes umfasst.

**30.** Vorrichtung nach Anspruch 29, wobei
das dritte und vierte Magnetfeld im Behandlungsbereich im wesentlichen parallel oder antiparallel verlaufen.

**31.** Vorrichtung nach einem der Ansprüche 29 bis 30, wobei das dritte Magnetfeld im Behandlungsbereich eine Stärke von 0,5 Gauss bis 500 Gauss, insbesondere 10 bis 50 Gauss, insbesondere im Bereich von 23 Gauss aufweist.

**32.** Vorrichtung nach einem der Ansprüche 29 bis 31, wobei das vierte Magnetfeld im Behandlungsbereich von 0 Gauss bis $\pm 5$ Gauss, insbesondere von 0 bis $\pm 2$ Gauss, insbesondere von 0 Gauss bis $\pm 0,5$ Gauss variierbar ist.

**33.** Vorrichtung nach einem der Ansprüche 16 bis 32, umfassend Mittel zur Änderung der Frequenz des zweiten Magnetfeldes, insbesondere zwischen 1 kHz und 1 Mhz, insbesondere zwischen 2 kHz und 200 kHz und insbesondere zwischen 90 und 110 kHz.

**34.** Vorrichtung nach Anspruch 33, wobei
die Richtung der makroskopischen Magnetisierung mittels der Veränderung der Frequenz des zweiten Magnetfeldes veränderbar ist.

**37.** Verfahren zur therapeutischen Behandlung mit magnetischen Feldern für die Behandlung von Tieren in einem Behandlungsbereich, in welchem Gewebe zumindest eines Körperbereichs eines zu behandelnden Patienten angeordnet wird, insbesondere mit einer Vorrichtung gemäß einem der vorstehenden Ansprüche, wobei
ein erstes und zweites Magnetfeld in dem Behandlungsbereich erzeugt werden, welche Magnetfelder sich in dem Behandlungsbereich zu einem Behandlungsfeld überlagern,
die in dem Gewebe enthaltenen Elemente und/oder Verbindungen in dem Behandlungsfeld zumindest eine Spinresonanzfrequenz aufweisen,
das zweite Magnetfeld ein magnetisches Wechselfeld umfasst, welches zumindest zeitweise eine Frequenz aufweist, welche der Spinresonanzfrequenz entspricht, das Behandlungsfeld mittels einer Veränderung zumindest eines der beiden ersten und zweiten Magnetfelder zeitlich veränderbar ist und
mittels der zeitlichen Veränderung des Behandlungsfeldes die Ausrichtung der Spins verändert wird.

Fig. 1a

Fig. 1b

Fig. 2

Fig. 3

Fig. 4

Fig. 5

90° Puls    180°Puls    Echo

Fig. 6

Echo, B = 23,4Gs

## Fig. 7

Tek   ⎍   ● Stop    M Pos: 33.70ms    CURSOR

Type
Time

Source
CH1

Delta
840.0μs
1.190kHz

Cursor 1
32.64ms

Cursor 2
33.48ms

CH1 200mV   CH2 10.0VBw   M 1.00ms    Ext ∕ 192mV

Echo, B = 23,2 Gs

## Fig. 8

Tek   ⎍   ● Stop    M Pos: 33.70ms    CURSOR

Type
Time

Source
CH1

Delta
840.0μs
1.190kHz

Cursor 1
32.72ms

Cursor 2
33.56ms

CH1 200mV   CH2 10.0VBw   M 1.00ms    Ext ∕ 192mV

Echo, B = 23,8 Gs

## Fig. 9

## Fig. 10a

$t = t_0$

## Fig. 10b

$t = t_1 > t_0$

## Fig. 10c

$t = t_2 > t_1$

Fig. 11

Fig. 12

## Fig. 13a

## Fig. 13b

Fig. 14

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 4026173
- WO 9966986 A

**In der Beschreibung aufgeführte Nicht-Patentliteratur**

- Orthopädische Praxis. August 2000, vol. 36, 510-515
- **FRITZ LECHNER.** Elektrostimulation und Magnetfeldtherapie. Anwendung, Ergebnisse und Qualitätssicherung. 1989
- **CHRISTIAN THUILE.** Das große Buch der Magnetfeldtherapie. Linz, 1997